# EUROPÄISCHE PATENTSCHRIFT

(11) **EP 1 366 730 B1**
(45) Veröffentlichungstag und Bekanntmachung des Hinweises auf die Patenterteilung: **26.09.2007**
(21) Anmeldenummer: 03008622.7
(22) Anmeldetag: 15.04.2003
(51) Int. Cl.: A61F 2/82

(54) **Selbstexpandierbarer Stent**
Self-expanding stent
Stent auto-expansible

(30) Priorität: 27.04.2002 DE 10219014
(43) Veröffentlichungstag der Anmeldung: 03.12.2003
(73) Patentinhaber: Boston Scientific Limited, an Irish company, Barbados, West Indies (BB)
(72) Erfinder: Schmitt, Klaus, 73630 Remshalden (DE)
(74) Vertreter: Vossius & Partner

(56) Entgegenhaltungen:
- EP-A- 0 621 015
- WO-A-97/36556
- US-A- 5 282 860
- US-A- 5 628 788
- US-A- 5 645 559
- US-A- 5 749 919
- US-A- 5 897 587
- US-A- 5 897 589
- US-A- 6 019 779
- US-B1- 6 264 684

## Beschreibung

Die Erfindung betrifft einen selbstexpandierbaren Stent zur transluminalen Implantation in ein Hohlorgan zur Schienung und/oder zum Offenhalten eines Hohlorgans, bestehend aus einer ersten elastischen tubulären Schicht auf der mehrere erste wendelförmige Filamente aufliegen, wobei die mehreren ersten wendelförmigen Filamente von einer zweiten elastischen tubulären Schicht überzogen sind, und wobei die zweite elastische tubuläre Schicht zumindest an ihren Enden mit der ersten elastischen tubulären Schicht materialschlüssig verbunden ist und die wendelförmigen Filamente lagefixiert. Mit Hilfe eines selbstexpandierbaren Stents können verengte Hohlorgane geschient und/oder offengehalten werden.

Ein derartiger Stent ist durch die Druckschrift WO 97 36556 A bekannt geworden.

Mit dem bekannten Stent können Gefäßverengungen nicht nur radial aufgeweitet sondern auch geschient bzw. offengehalten werden. In einem verjüngten und gelängten Zustand wird der Stent in ein Hohlorgan eingeführt und an der verengten Stelle des Hohlorgans radial expandiert, damit das Hohlorgan an dieser Stelle möglichst dauerhaft sein ursprüngliches Lumen einnimmt. Zur Selbstexpansion sind Geflechte bekannt, die man zu Stents verarbeitet. Die Geflechte können gegen ihre belastungsfreie Ausgangsstruktur gelängt werden. Gibt man die Längung bzw. eine radiale Deformation auf, so kehren diese bekannten Stents in ihren Ausgangszustand zurück, indem sie sich radial weiten. Diesen Effekt nützt man zur Weitung von Stenosen in Hohlorganen, so dass deren Funktion möglichst nicht beeinträchtigt wird.

Aufgabe der Erfindung ist es, einen Stent zu entwickeln, der auf ein breites Ansprechverhalten mit einfachen Mitteln ausgelegt werden kann, eine verbesserte Flexibilität aufweist und sicher, dauerhaft und unverrückbar in einem Hohlorgan zu platzieren ist.

Erfindungsgemäß wird die Aufgabe dadurch gelöst, dass alle in der Ebene auf der ersten tubulären Schicht angeordneten Filamente phasenverschoben jeweils beabstandet und berührungsfrei bezüglich anderer Filamente angeordnet sind.

Der erfindungsgemäße selbstexpandierbare Stent hat damit den wesentlichen Vorteil, dass er auf ein Geflecht verzichten kann und dennoch dauerhaft und sicher Abschnitte eines Hohlorgans offenhält. Auf eine erste elastische tubuläre Schicht, die zum Beispiel aus Silikon besteht, und deren Innenoberfläche glatt und/oder hydrophil beschichtet sein kann, werden auf deren Außenoberfläche erste wendelförmige Filamente aufgebracht, die von einer zweiten elastischen tubulären Schicht, beispielsweise einer Silikonschicht, überzogen sind, die die Wendel auf der ersten elastischen tubulären Schicht dauerhaft lagefixiert hält. Die Wendel dienen zur Armierung der elastischen Kunststoffschichten, und es versteht sich, dass die erste und zweite elastische tubuläre Schicht aus jedwedem medizinisch verträglichen Polymer hergestellt sein kann. Die Wendel stellen in einfachster Form sicher, dass auch unter einem gewissen radialen Druck auf die Außenoberfläche des Stents der erfindungsgemäße Stent nicht kollabiert und den gewünschten Abschnitt eines Hohlorgans dauerhaft stützt und offenhält.

Die erste und zweite elastische tubuläre Schicht sind zumindest an den freien Enden des erfindungsgemäßen Stents miteinander materialschlüssig verbunden, damit die zwischen den Schichten gelagerten Wendel in jedem Zustand eine Schichtumhüllung aufweisen. Durch eine große Beweglichkeit der Wendel zwischen den Schichten kann die Charakteristik des erfindungsgemäßen Stents über die federnden Eigenschaften der Wendel eingestellt werden.

Durch die Verwendung mehrerer erster wendelförmiger Filamente, die phasenverschoben und jeweils beabstandet bezüglich anderer Filamente in einer Ebene auf der ersten elastischen tubulären Schicht aufliegen, werden die Kräfte zur Expansion eines erfindungsgemäßen Stents verstärkt. Alle ersten wendelförmigen Elemente liegen also in einer Ebene (Zylindermantelfläche). Die phasenverschobenen ersten wendelförmigen Filamente berühren sich nicht und sind vollkommen von der zweiten elastischen tubulären Schicht umschlossen, die wiederum mit der ersten elastischen tubulären Schicht einen Materialverbund eingeht. Gewünschte Rückstellkräfte können über mehrere phasenverschobene erste wendelförmige Filamente definiert eingestellt werden.

Werden die erste und zweite elastische tubuläre Schicht materialschlüssig über die gesamte Länge des erfindungsgemäßen Stents miteinander verbunden, so ist sichergestellt, dass sich die Wendel im Materialverbund nicht aufgrund eines lokalen Drucks punktuell verschieben lässt. Der erfindungsgemäße Stent bildet eine Einheit, die sich aus der gezielten Zusammenwirkung der beiden elastischen tubulären Schichten mit der Wendel ergibt.

Ist die zweite elastische tubuläre Schicht im Querschnitt dünner als das erste oder die mehreren ersten wendelförmigen Filamente im Querschnitt, so stehen die Strukturen der Wendel über die Außenoberfläche des erfindungsgemäßen Stents vor, und es stellt sich eine Oberflächenstruktur (Topographie) ein, die den erfindungsgemäßen Stent möglichst positionsstabil an der gewünschten Stelle des Hohlorgans hält.

Sind mehrere erste wendelförmige Filamente vorgesehen, so können diese untereinander unterschiedliche Querschnitte und/oder Durchmesser oder Höhen /Breitenmaße aufweisen.

Dies hat den Vorteil, dass das Expansionsverhalten eines erfindungsgemäßen Stents mit einfachsten Änderungen durch eine Querschnittsveränderung der Wendel beeinflusst werden kann. Kreisförmige, ovale, dreieckförmige, rechteckige und quadratische Querschnittsformen einer Wendel sind denkbar und unterschiedlichste Querschnitte lassen sich kombinieren.

Bevorzugt ist, dass mindestens ein wendelförmiges Filament aus Kunststoff, Metall oder Karbon hergestellt ist. Die wendelförmigen Filamente haben darüber hinaus den Vorteil, dass sich unterschiedliche Materialien in einem Materialverbund kombinieren lassen, aus dem kein Geflecht, Netz oder Gewebe hergestellt werden kann. Bei dem erfindungsgemäßen Stent liegen die wendelförmigen Filamente beabstandet voneinander im Materialverbund der ersten und zweiten elastischen tubulären Schicht und kreuzen sich nicht. Wendeln aus unterschiedlichen Materialien lassen sich kombinieren und in einer Ebene verlegen.

Die erste und zweite elastische tubuläre Schicht weist in einer weiteren bevorzugten Ausführungsform des erfindungsgemäßen Stents ein unterschiedliches elastisches Verhalten auf, und die Schichten bestehen aus einem oder mehreren Polymeren. Dies hat den Vorteil, dass man das Expansionsverhalten des erfindungsgemäßen Stents auch über die erste und zweite elastische tubuläre Schicht beeinflussen und definiert steuern kann. Von Bedeutung sind hier die Materialschichten selbst und deren Eigenschaften unter dem Einfluss von Druck, Temperatur und Feuchtigkeit.

Liegen auf den ersten wendelförmigen Filamenten mindestens ein zweites wendelförmiges Filament außerhalb einer von den ersten wendelförmigen Filamenten aufgespannten Ebene auf und ist dieses zweite wendelförmige Filament mit einer dritten elastischen tubulären Schicht aus einem Polymer überzogen, die mit der zweiten elastischen tubulären Schicht verbunden ist, so lassen sich noch weitere Eigenschaften an dem erfindungsgemäßen Stent ausbilden, die mit Geflechten nicht erreicht werden können. Das zweite wendelförmige Element liegt in einer zweiten Ebene über dem ersten wendelförmigen Filament und kann unterschiedliche Materialeigenschaften gegenüber den ersten wendelförmigen Filamenten aufweisen. Das zweite wendelförmige Filament kann mehr oder weniger stark von den ersten wendelförmigen Filamenten beabstandet sein, und somit verändert sich das Selbstexpansionsverhalten des erfindungsgemäßen Stents vollkommen gegenüber bekannten selbstexpandierenden Stents. Wird der erfindungsgemäße Stent gelängt, so werden wendelförmige Filamente mit einem unterschiedlichen Durchmesser zu einer zentralen longitudinal verlaufenden Stentachse unterschiedlich stark gedehnt, so dass Materialverwerfungen gezielt provoziert werden können. Dieses Spannungspotential kann für ein gewünschtes Expansionsverhalten und für eine sichere Positionierung genutzt werden.

In einer weiteren bevorzugten Ausführungsform liegen mehrere zweite wendelförmige Filamente phasenverschoben angeordnet auf dem mindestens einen wendelförmigen Filament auf. Dies hat den Vorteil, dass der erfindungsgemäße Stent mit einer mehr oder weniger stark verdichteten Lage von zweiten wendelförmigen Filamenten in einer Ebene nicht nur verstärkt versteift oder beweglicher ausgestaltet werden kann, sondern seine Außenoberflächenbeschaffenheit kann anwendungsbezogen ausgelegt werden.

Eine dazu alternative Ausführungsform kann dadurch geschaffen werden, wenn das mindestens eine zweite wendelförmige Filament zu den ersten wendelförmigen Elementen eine entgegengesetzte Windungsrichtung aufweist. Mit dieser vielfach besonders geeigneten Ausführungsform wird der erfindungsgemäße Stent beim Längen bzw. Strecken torquiert, und diese Torquierung ist reversibel, so dass sich bei einem Expansionsvorgang die Vortorquierung zurückstellt und sich der erfindungsgemäße Stent schraubenartig in die Innenoberfläche eines Hohlorgans eindreht oder sich dort anlegt.

Weitere Vorteile ergeben sich für einen erfindungsgemäßen Stent, wenn die mehreren ersten und/oder zweiten wendelförmigen Filamente unterschiedliche Steigungswinkel aufweisen. Mit derartigen Ausgestaltungen lassen sich vielfältige Stent-Designs erreichen, die sich durch unterschiedlichste mechanische Eigenschaften auszeichnen. Unterschiedlichste Stauchungsgrade und Längendehnungen in unterschiedlichen Ebenen mit einem unterschiedlich starken Ausmaß lassen sich sowohl beim Längen wie auch bei der Expansion eines Stents ausbilden.

Unterstützt werden die Expansionseigenschaften des erfindungsgemäßen Stents noch dadurch, wenn die dritte elastische tubuläre Schicht eine zur zweiten elastischen tubulären Schicht unterschiedliche Dicke und/oder ein unterschiedliches elastisches Verhalten aufweist. Die dritte elastische tubuläre Schicht stellt einen weiteren frei beeinflussbaren Parameter dar, über den das Selbstexpansionsverhalten des erfindungsgemäßen Stents signifikant beeinflusst werden kann.

Mit dem erfindungsgemäßen Stent ist ein selbstexpandierender Stent geschaffen, der über zahlreiche steuerbare Parameter in einfachster Weise in seinen Bewegungseigenschaften beeinflusst werden kann. So kann ein mehrlagiger selbstexpandierender Stent mit Vorshortening und Vortorquierung geschaffen werden, die Innenoberfläche des erfindungsgemäßen Stents kann anwendungsbezogen sehr glatt gehalten werden, und ein erstes Bündel von wendelförmigen Filamenten beeinflusst die Innenoberfläche des erfindungsgemäßen Stents nicht, weil dieses Bündel von wendelförmigen Filamenten auf der Außenoberfläche der ersten elastischen tubulären Schicht aufliegt und sich nicht auf die Oberflächenbeschaffenheit der Innenoberfläche auswirkt beziehungsweise durchdrückt. Die ersten und/oder zweiten wendelförmigen Filamente können gebündelt mehr oder weniger stark aneinanderliegen oder voneinander beabstandet sein. Sie kreuzen sich jedoch nicht. Die ersten und/oder zweiten Wendeln können unterschiedliche Steigungen aufweisen, und unterschiedlichste Materialien von Wendeln lassen sich in jeder Ebene kombinieren. Die Filamente können unterschiedlich stark ausgelegt sein, und die tubulären Schichten können ebenfalls eine unterschiedliche Dicke aufweisen. Die ersten und zweiten wendelförmigen Filamente können unterschiedlich weit voneinander beabstandet sein (in radialer Richtung gesehen), und die ersten wendelförmigen Filamente können stärker in eine elastische Schicht eingebettet sein als die zweiten wendelförmigen Filamente. Sind die zweiten wendelförmigen Filamente weniger stark fixiert in die dritte elastische tubuläre Schicht eingebettet, so sind diese zweiten wendelförmigen Filamente leichter herausprodrudierbar, und diese zweiten wendelformigen Filamente können die Außenoberflächenstruktur des erfindungsgemäßen Stents signifikant prägen.

Weitere Vorteile der Erfindung ergeben sich aus der Figurenbeschreibung, bei der einzelne Merkmale stark schematisiert dargestellt sind. Die Darstellungen in den Figuren sind nicht maßstäblich zu verstehen, sondern haben vielmehr beispielhaften Charakter. Es zeigt:
- Fig. 1: einen erfindungsgemäßen Stent in räumlicher Darstellung mit phasenverschobenen ersten wendelförmigen Filementen;
- Fig. 2: eine Seitenansicht eines weiteren erfindungsgemäßen Stents mit phasenverschobenen ersten wendelförmigen Filamenten;
- Fig. 3: eine Ausführungsform eines erfindungsgemäßen Stents in Seitenansicht mit ersten und zweiten wendelförmigen Filamenten in unterschiedlichen Ebenen und mit entgegengesetzter Windungsrichtung zwischen dem ersten und zweiten wendelförmigen Filament;
- Fig. 4: eine weitere Ausführungsform eines erfindungsgemäßen selbstexpandierbaren Stents im Querschnitt mit ersten phasenverschobenen wendelförmigen Filamenten und zweiten phasenverschobenen wendelförmigen Filamenten mit entgegengesetzter Windungsrichtung.

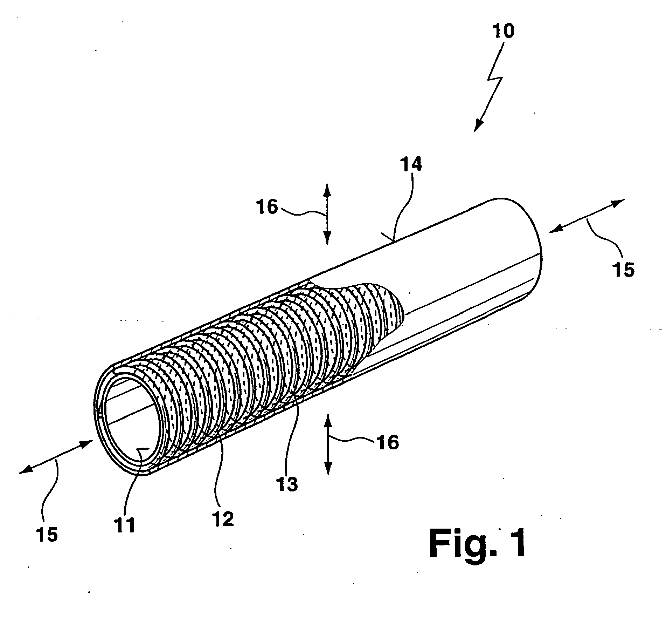

**Fig. 1** zeigt mit 10 einen Stent, der sich aus einer ersten elastischen tubulären Schicht 11, einem ersten wendelförmigen Filament 12, einem phasenverschobenen ersten wendelförmigen Filament 13 und einer zweiten elastischen tubulären Schicht 14 zusammensetzt. Die ersten wendelförmigen Filamente 12, 13 liegen in einer Ebene voneinander beabstandet und kreuzen sich nicht. Weitere erste wendelförmige Filamente können in den Stent 10 eingebunden sein, sofern andere Stent-Designs gewünscht werden. Wird der erfindungsgemäße Stent 10 in Pfeilrichtungen 15 gelängt, so verjüngt sich der Stent 10 im Außenumfang. Wird die unter Zwang geschaffene Längung des Stents 10 aufgegeben, so findet eine Selbstexpansion des Stents 10 statt, und der Stent 10 vergrößert sich in radialer Richtung in Pfeilrichtung 16, indem er sich verkürzt (zusammenzieht) und ein größeres Lumen begrenzt als im verjüngten, gelängten Zustand. Auf eine im Tauchverfahren hergestellte erste elastische tubuläre Schicht 11 werden mehrere wendelförmige Filamente wendelförmig und phasenverschoben zueinander auf die erste elastische tubuläre Schicht 11 aufgelegt, und über einen weiteren Materialübertauch werden die ersten wendelförmigen Filamente 12, 13 in eine Polymermatrix, zum Beispiel aus Silikon, fest eingebettet, die sich mit der ersten elastischen tubulären Schicht 11 materialschlüssig verbindet. Die zweite elastische tubuläre Schicht 14 kann aus demselben Polymermaterial geschaffen sein wie die erste elastische tubuläre Schicht 11. Beide Schichten 11, 14 können unterschiedliche Dicken aufweisen.

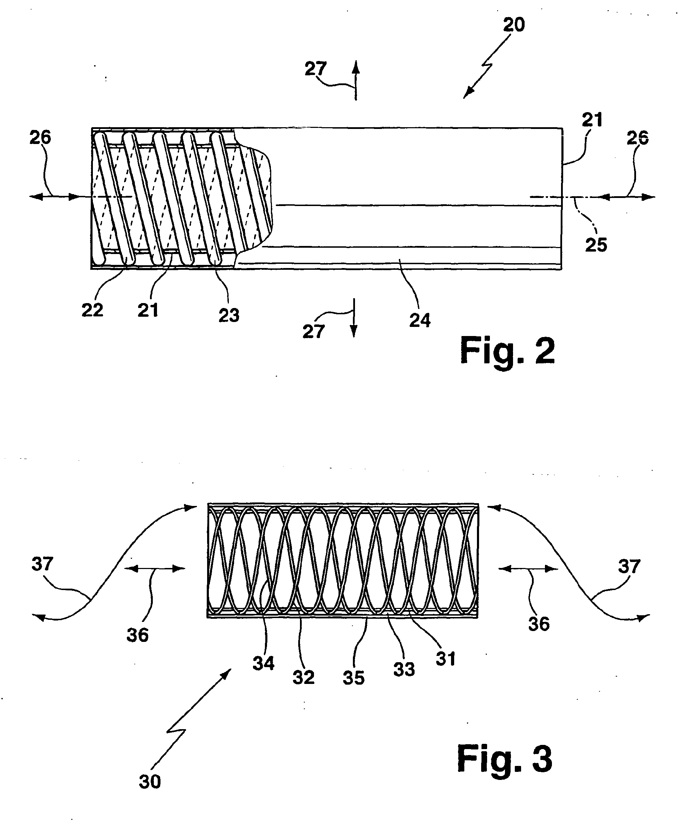

**Fig. 2** zeigt einen weiteren erfindungsgemäßen Stent 20 in einer Seitenansicht. Auf einer ersten elastischen tubulären Schicht 21 ist ein erstes wendelförmiges Filament 22 aus Metall, Karbon oder Kunststoff aufgelegt und dazu phasenverschoben ein weiteres erstes wendelförmiges Filament 23. Die ersten wendelförmigen Filamente 22, 23 sind mit einer zweiten elastischen tubulären Schicht 24 vollkommen überzogen, die mit der ersten elastischen tubulären Schicht 21 einen Materialverbund eingeht. Von einer zentralen Achse 25 des erfindungsgemäßen Stents 20 sind die ersten wendelförmigen Filamente 22, 23 gleich weit beabstandet, so dass sie in einer Ebene liegen. In Pfeilrichtungen 26 kann der Stent 20 gelängt bzw. verkürzt werden. Der Stent 20 ist derart ausgelegt, dass er sich selbstexpandierend zusammenzieht und sich dabei in radialer Richtung vergrößert. In Pfeilrichtungen 27 kann sich der Stent 20 verjüngen bzw. expandieren. Die elastischen tubulären Schichten 21, 24 können unterschiedliche Materialeigenschaften aufweisen, sie können unterschiedlich gefärbt sein und eine oder beide Schichten können röntgenschattengebend ausgelegt sein.

**Fig. 3** zeigt eine besonders vorteilhafte Ausführungsform eines erfindungsgemäßen Stents 30, der auf einer ersten elastischen tubulären Schicht 31 ein erstes wendelförmiges Filament 32 aufweist. Das erste wendelförmige Filament 32 ist über eine zweite elastische tubuläre Schicht 33 an die erste elastische tubuläre Schicht 31 gebunden und dort lagefixiert. Auf dem ersten wendelförmigen Filament 32 und somit auf der zweiten elastischen tubulären Schicht 33 ist außerhalb der Ebene des ersten wendelförmigen Filaments 32 ein zweites wendelförmiges Filament 34 aufgelegt, das das erste wendelförmige Filament 32 radial beabstandet überdeckt. Lagefixiert ist das zweite wendelförmige Filament 34 über eine dritte elastische tubuläre Schicht 35, die fest mit der zweiten elastischen tubulären Schicht 33 verbunden ist. Das zweite wendelförmige Filament 34 weist gegenüber dem ersten wendelförmigen Filament 32 eine entgegengesetzte Windungsrichtung auf, so dass beim Längen des Stents 30 eine Verwindung des Stents 30 stattfindet. Wird der Stent 30 in Pfeilrichtungen 36 gelängt, so findet gleichzeitig eine Verdrehung und Verjüngung in Pfeilrichtungen 37 statt (Vortorquierung beim Strecken). Der Stent 30 besteht aus mehreren Lagen, und die wendelförmigen Filamente 32, 34 sind in unterschiedlichen Ebenen im Stent 30 angeordnet. Neben den ersten und zweiten wendelförmigen Filamenten 32, 34 können in jeder Ebene noch weitere phasenverschobene erste und zweite wendelförmige Filamente in die Polymermatrix eingebettet sein.

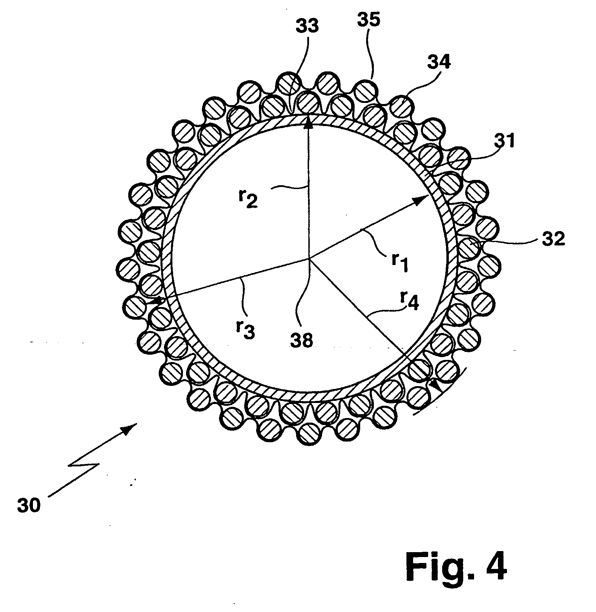

**Fig. 4** zeigt einen Querschnitt durch einen Stent im expandierten Zustand mit mehreren ersten und zweiten wendelförmigen Filamenten. Von einer zentralen Achse 38 in Längsrichtung des Stents gesehen, ist die erste elastische tubuläre Schicht 31 im Radius r₁ von der zentralen Achse 38 beabstandet. Auf der Außenoberfläche der ersten elastischen tubulären Schicht 31 sind die ersten wendelförmigen Filamente 32 angeordnet, die einen Abstand r₂ von der zentralen Achse 38 aufweisen. Ummantelt sind die ersten wendelförmigen Filamente 32 von einer zweiten elastischen tubulären Schicht 33. Auf den ersten wendelförmigen Filamenten 32 liegen in entgegengesetzter Windungsrichtung ausgerichtet zweite wendelförmige Filamente 34 in einem Abstand r₃ von der zentralen Achse 38. Im expandierten Zustand des Stents ist der Außenradius mit r₄ angegeben, der von einer dritten elastischen tubulären Schicht 35 begrenzt ist, die die zweiten wendelförmigen Filamente 34 ummanteln. r₁ ist < als r₂, r₂ ist < als r₃ und r₃ ist < als r₄.

Bei einem erfindungsgemäßen Stent 10 sind auf einer ersten tubulären Schicht 11 wendelförmige Filamente 12, 13 angeordnet. Die wendelförmigen Filamente 12, 13 sind zueinander phasenverschoben derart, dass sie sich nicht berühren und in einer Ebene liegen. Die wendelförmigen Filamente 12, 13 sind von einer zweiten elastischen tubulären Schicht 14 vollkommen überzogen, die mit der ersten elastischen tubulären Schicht 11 verbunden ist. Auf der zweiten elastischen tubulären Schicht 14 können weitere wendelförmige Filamente angeordnet sein. Der erfindungsgemäße Stent lässt sich zum Einbringen in ein Hohlorgan längen und ist selbstexpandierbar.

## Patentansprüche

1. Selbstexpandierbarer Stent (10; 20; 30) zur Schienung und/oder zum Offenhalten eines Hohlorgans, bestehend aus einer ersten elastischen tubulären Schicht (11; 21; 31) auf der mehrere erste wendelförmige Filamente (12,13; 22, 23; 32) in einer Ebene über der ersten elastischen tubulären Schicht (11; 21; 31) aufliegen, wobei die mehreren ersten wendelförmigen Filamente (12, 13; 22, 23; 32) von einer zweiten elastischen tubulären Schicht (14; 24; 33) überzogen sind, und wobei die zweite elastische tubuläre Schicht (14; 24; 33) zumindest an ihren Enden mit der ersten elastischen tubulären Schicht (11; 21; 31) materialschlüssig verbunden ist und die wendelförmigen Filamente (12, 13; 22, 23; 32) lagefixiert,
**dadurch gekennzeichnet, dass**
alle in der Ebene auf der ersten tubulären Schicht (14) angeordneten Filamente (12, 13; 22, 23; 32) phasenverschoben jeweils beabstandet und berührungsfrei bezüglich anderer Filamente angeordnet sind; und
über den ersten wendelförmigen Filamenten (32) mindestens ein zweites wendelförmiges Filament (34) außerhalb einer von den ersten wendelförmigen Filamenten (32) aufgespannten Ebene vorgesehen ist.

2. Stent nach Anspruch 1, **dadurch gekennzeichnet, dass** die erste und zweite elastische tubuläre Schicht (11, 14; 21, 24; 31, 33) über die gesamte Länge des Stents (10; 20; 30) materialschlüssig miteinander verbunden sind.

3. Stent nach Anspruch 1 oder 2, **dadurch gekennzeichnet, dass** die zweite elastische tubuläre Schicht (14; 24; 33) im Querschnitt dünner ist als das erste oder die mehreren ersten wendelförmigen Filamente (12, 13; 22, 23; 32) im Querschnitt.

4. Stent nach Anspruch 1, **dadurch gekennzeichnet, dass** die mehreren ersten wendelförmigen Filamente (12, 13; 22, 23; 32) unterschiedliche Querschnitte und/oder Durchmesser oder Höhen-/Breitenmaße aufweisen.

5. Stent nach Anspruch 1, **dadurch gekennzeichnet, dass** die erste und zweite elastische tubuläre Schicht (11, 14; 21, 24; 31, 33) ein unterschiedliches elastisches Verhalten aufweisen und aus einem oder mehreren Polymeren bestehen.

6. Stent nach Anspruch 1, **dadurch gekennzeichnet, dass** das mindestens eine zweite wendelförmige Filament (34) mit einer dritten elastischen tubulären aus einem Polymer bestehenden Schicht (35) überzogen ist, die mit der zweiten elastischen tubulären Schicht (33) verbunden ist.

7. Stent nach Anspruch 1, **dadurch gekennzeichnet, dass** mehrere zweite wendelförmige Filamente (34) zueinander phasenverschoben angeordnet auf den ersten wendelförmigen Filamenten (32) aufliegen.

8. Stent nach Anspruch 1, **dadurch gekennzeichnet, dass** das mindestens eine zweite wendelförmige Filament (34) eine zu den ersten wendelförmigen Filamenten (32) entgegengesetzte Windungsrichtung aufweist.

## Claims

1. Self-expanding stent (10; 20; 30) for splinting and/or keeping open a hollow organ, consisting of a first elastic tubular layer (11; 21; 31) on which several first helical filaments (12, 13; 22, 23; 32) are disposed in one plane over the first elastic tubular layer (11; 21; 31), wherein the several first helical filaments (12, 13; 22, 23; 32) are coated by a second elastic tubular layer (14; 24; 33), and wherein the second elastic tubular layer (14; 24; 33) is connected at least at its ends with the first elastic tubular layer (11; 21; 31) in a material-bonding fashion and fixes the position of the helical filaments (12, 13; 22, 23; 32),
**characterized in that**
all filaments (12, 13; 22, 23; 32) which are disposed in the plane on the first tubular layer (14) are disposed in a phase-shifted and mutually distanced fashion and do not touch another relative to other filaments, and
at least one second helical filament (34) is disposed over the first helical filaments (32) outside of a plane spanned by the first helical filaments (32).

2. Stent according to claim 1, **characterized in that** the first and second elastic tubular layers (11, 14; 21, 24; 31, 33) are connected to another over the entire length of the stent (10; 20; 30) in a material-bonding fashion.

3. Stent according to claim 1 or 2, **characterized in that** the cross-section of the second elastic tubular layer (14; 24; 33) is thinner than the cross-section of the first or several first helical filaments (12, 13; 22, 23; 32).

4. Stent according to claim 1, **characterized in that** the several first helical filaments (12, 13; 22, 23; 32) have different cross-sections and/or diameters or heights/widths.

5. Stent according to claim 1, **characterized in that** the first and second elastic tubular layers (11, 14; 21, 24; 31, 33) have different elastic properties and consist of one or more polymers.

6. Stent according to claim 1, **characterized in that** the at least one second helical filament (34) is coated with a third elastic tubular layer (35) consisting of a polymer which is connected to the second elastic tubular layer (33).

7. Stent according to claim 1, **characterized in that** several second helical filaments (34) are disposed in a mutually phase-shifted manner on the first helical filaments (32).

8. Stent according to claim 1, **characterized in that** the at lest one second helical filament (34) has a winding direction which is opposite to the first helical filaments (32).

## Revendications

1. Stent auto-expansible (10 ; 20 ; 30) permettant d'éclisser et/ou de maintenir ouvert un organe creux, constitué d'une première couche tubulaire élastique (11 ; 21 ; 31), sur laquelle reposent plusieurs premiers filaments hélicoïdaux (12, 13 ; 22, 23 ; 32) dans un plan au-dessus de la première couche tubulaire élastique (11 ; 21 ; 31), dans lequel la pluralité de premiers filaments hélicoïdaux (12, 13; 22, 23 ; 32) sont recouverts d'une deuxième couche tubulaire élastique (14 ; 24 ; 33); et dans lequel la deuxième couche tubulaire élastique (14 ; 24 ; 33) est reliée au moins à ses extrémités à la première couche tubulaire élastique (11 ; 21 ; ;31) d'une manière liant les matières et fixe en position les filaments hélicoïdaux (12, 13 ; 22, 23 ; 32),
**caractérisé en ce que**
tous les filaments (12, 13 ; 22, 23 ; 32) disposés dans le plan sur la première couche tubulaire (14) sont espacés respectivement de manière décalée et sont disposés sans contact par rapport à d'autres filaments ; et
au moins un deuxième filament hélicoïdal (34) est prévu au-dessus des premiers filaments hélicoïdaux (32) en dehors d'un plan défini par les premiers filaments (32) hélicoïdaux.

2. Stent selon la revendication 1, **caractérisé en ce que** la première et deuxième couche tubulaire élastique (11, 14 ; 21, 24 ; 31, 33) sont reliées l'une à l'autre d'une manière liant les matières sur toute la longueur du stent (10 ; 20 ; 30).

3. Stent selon la revendication 1 ou 2, **caractérisé en ce que** la deuxième couche tubulaire élastique (14 ; 24 ; 33) dispose d'une section plus fine que la section du premier ou de la pluralité de premiers filaments hélicoïdaux (12, 13 ; 22, 23 ; 32).

4. Stent selon la revendication 1, **caractérisé en ce que** la pluralité de premiers filaments hélicoïdaux (12, 13 ; 22, 23 ; 32) présente des sections et/ou diamètres ou hauteur/largeur différents.

5. Stent selon la revendication 1, **caractérisé en ce que** la première et deuxième couche tubulaire élastique (11, 14 ; 21, 24 ; 31, 33) présentent un comportement élastique différent et sont constituées d'un ou de plusieurs polymères.

6. Stent selon la revendication 1, **caractérisé en ce que** l'au moins un deuxième filament hélicoïdal (34) est recouvert d'une troisième couche tubulaire élastique (35) en polymère, qui est reliée à la deuxième couche tubulaire élastique (33).

7. Stent selon la revendication 1, **caractérisé en ce que** plusieurs deuxièmes filaments hélicoïdaux (34) sont disposés de manière décalée les uns par rapport aux autres sur les premiers filaments hélicoïdaux (32).

8. Stent selon la revendication 1, **caractérisé en ce que** l'au moins un deuxième filament hélicoïdal (34) présente un sens d'enroulement opposé aux premiers filaments hélicoïdaux (32).
